Europäisches Patentamt

European Patent Office

Office européen des brevets

(19)

(11) Publication number: **0 363 117 A1**

(12) **EUROPEAN PATENT APPLICATION**

(21) Application number: **89310045.3**

(22) Date of filing: **02.10.89**

(51) Int. Cl.⁵: **A61B 5/021 , A61B 5/028 , G01L 19/00**

(30) Priority: **06.10.88 US 254501**

(43) Date of publication of application:
**11.04.90 Bulletin 90/15**

(84) Designated Contracting States:
**DE FR GB**

(71) Applicant: **BAXTER INTERNATIONAL INC.**
**One Baxter Parkway**
**Deerfield, IL 60015(US)**

(72) Inventor: **Ganz, William**
**1550 Rising Glen Road**
**Los Angeles, CA 90069(US)**
Inventor: **Swan, Harold J.**
**1075 Wallace Ridge**
**Beverly Hills, CA 90210(US)**
Inventor: **Woodgrift, Randel**
**9 Precipice**
**Laguna Niguel, CA 92677(US)**

(74) Representative: **MacGregor, Gordon et al**
**ERIC POTTER & CLARKSON 14 Oxford Street**
**Nottingham, NG1 5BP(GB)**

(54) **A position-monitoring flow-directed catheter and method.**

㊗ A balloon-tipped, flow-directed catheter (25) with a position-monitoring port (29) is adapted to be positioned in the outflow tract of the right ventricle (13) proximal to the pulmonic valve (18) of the heart (H), which in most adult patients would be approximately 8 cm from the distal tip (26) of the catheter. By measuring the pressure at the position-monitoring port (29), the position of the catheter tip (26) and the balloon (27) in the pulmonary artery (17) can be monitored, and checked before inflating the balloon to measure pulmonary wedge pressure at the distal port. A right ventricular pressure reading indicates that the balloon is in a proper position in the pulmonary artery for measuring wedge pressure. A pulmonary artery pressure reading indicates that the position-monitoring port has crossed the pulmonic valve and that the balloon has migrated distally to a position that may not be safe for inflating the balloon to measure wedge pressure. In such a case, the catheter should be gradually withdrawn until a right ventricular pressure is obtained.

FIG. 1

# A POSITION-MONITORING FLOW-DIRECTED CATHETER AND METHOD

### Field of Invention

In general, the present invention relates to an intravascular catheter for cardiac monitoring and, more particularly, a balloon-tipped, flow-directed thermodilution catheter and a method for monitoring intracardiac and pulmonary pressures and cardiac output.

### Background of the Invention

Intravascular catheters are commonly used in cardiac monitoring. One such catheter, sometimes referred to as a flow-directed thermodilution catheter is used as a diagnostic tool for measuring intravascular pressures of the heart and for measuring cardiac output. The catheter is generally provided with three to four lumens, but may be provided with as many as six, including one lumen for inflating the balloon located at the distal catheter tip. A second lumen, the through lumen, extends the length of the catheter and has an opening at the distal catheter tip for measuring pressure. A third lumen extends along the length of the catheter and terminates at an opening, typically about 30 cm from the distal catheter tip, and is used for injecting or infusing solutions. The catheter also includes a thermistor which is typically positioned in a fourth lumen about 4 cm from the distal tip of the catheter. The catheter may also have fifth and sixth lumens with openings at the atrial and ventricular portions of the catheter for insertion of electrode probes for cardiac pacing.

In operation, the catheter is introduced into a central vein and advanced toward the right heart through the superior vena cava or the inferior vena cava. When the distal tip of the catheter is positioned within the vena cava near the junction of the right atrium, the balloon located at the tip is inflated through the inflation lumen. As the catheter is advanced further, the balloon is directed by the blood stream from the vena cava through the right atrium, right ventricle and into the main pulmonary artery. The catheter is advanced until the balloon is lodged in a branch of the pulmonary artery. In this position, referred to as the pulmonary wedge position, the inflated balloon seals off the inflow of blood through the artery, so that the pressure of the left atrium and left ventricle can be measured at the distal tip of the catheter. When the balloon is deflated, the pulmonary artery pressure is measured at the distal tip of the catheter. A pressure measuring apparatus, such as a pressure transducer, is coupled to the proximal port of the through lumen for measuring these pressures.

In addition, the cardiac output of the heart can be determined by injecting a chilled solution through the third lumen opening at the 30 cm location and measuring the resulting drop in blood temperature downstream at the site of the thermistor. The time course of temperature change is used to calculate the cardiac output in accordance with the thermodilution method.

The catheter may also have electrodes coiled around the tube at the atrial and ventricular portions of the catheter for cardiac pacing as described and disclosed in U.S. Patent No. 3,995,623, issued December 7, 1976, in the name of Blake et al, for a Multipurpose Flow-Directed Catheter.

The ideal position of the pulmonary artery catheter is when the tip of the catheter is in the main right or left pulmonary artery branch. In this position, an undistorted pulmonary artery pressure can be recorded. When the balloon at the tip is inflated, the distal tip of the catheter can move forward into a wedged position to measure left atrial and ventricular pressure. When the balloon is deflated, the tip of the catheter moves back into its original position, and a pulmonary artery pressure is recorded. Under these conditions, an undistorted wedge pressure is obtained safely without damage to the pulmonary artery wall.

However, after insertion into a patient, the catheter body warms up and tends to soften, causing the catheter tip to be gradually propelled forward by contractions of the heart into smaller pulmonary artery branches. When the balloon is inflated in a small branch, the wall of the pulmonary artery may be ruptured resulting in massive, sometimes fatal bleeding into the lungs. Inflation of the balloon in a small branch may also give a wrong wedge pressure value ("over wedge"). Even without inflation of the balloon at the distal tip, the catheter may advance into very small branches, obstructing the lumen of the artery, causing pulmonary infarction damage to the lungs.

U.S. Patent No. 4,696,304, issued September 29, 1987, in the name of Chin, for a Thermodilution Flow-Directed Catheter Assembly and Method, attempts to solve this problem by providing a catheter assembly comprising two catheters, an outer catheter having a lumen in which an inner catheter having second and third lumens is slidably disposed. An inflatable balloon is positioned at the distal end of the inner catheter and is in communication with the second lumen, the inflation lumen. The third lumen of the inner catheter is used for pressure measurements. In operation, the distal section of the catheter assembly is inserted into the heart of the patient in the conventional manner, with the inner catheter substantially retracted within the outer catheter. The balloon is inflated through the second lumen so that the catheter assembly tip will be guided by blood flow until the tip of the assembly is positioned in the pulmonary artery. The catheter assembly is then advanced until the balloon occludes a pulmonary artery (the wedged position).

Once the balloon is in the wedged position, pressure measurements may be made utilizing the third lumen. After the first wedge pressure measurement has been made the balloon is deflated and the catheter assembly is pulled back a small distance, typically about 7 cm, so that the distal tip of the inner catheter remains in the main pulmonary artery. The outer catheter is then stitched in place. Subsequent pressure measurements are made by reinflating the balloon and advancing the inner catheter through the outer catheter until the balloon occludes a pulmonary artery. After the wedge pressure measurement is taken, the balloon is deflated, and the inner catheter is retracted.

In this way, Chin attempts to solve the problem of distal migration of the balloon-tipped catheter and avoid pulmonary infarction, or pulmonary artery injury or rupture. The disadvantages of the Chin method are the delay between pressure measurements and the repeated repositioning of the inner catheter, with increased likelihood of infection and clotting. The present invention solves the same problem without those disadvantages.

## Objects and Summary of the Invention

It is therefore an object of the present invention to prevent injury or rupture of the pulmonary artery, that may be caused by inflation of the balloon of a flow-directed catheter after undetected distal migration of the balloon to the periphery of the pulmonary artery bed.

It is also an object of the present invention to prevent pulmonary infarction caused by occlusion of a smaller pulmonary artery branch following undetected distal migration of the deflated balloon.

It is a specific object of the present invention to detect distal migration of the balloon prior to inflation of the balloon to measure pulmonary wedge pressure.

Another specific object of the present invention is to continuously monitor, as needed, the position of the catheter tip and balloon by continuous monitoring of the pressure at a port in the catheter, positioned in the outflow tract of the right ventricle proximal to the pulmonic valve.

In general, the present invention provides a balloon-tipped, flow-directed intravascular catheter and a method for monitoring intracardiac and pulmonary pressures, cardiac output and blood oxygen saturation, for ventricular and atrial pacing, and for infusion of medication, that provides the user with means for monitoring the position of the balloon in the pulmonary artery and detects distal displacement of the catheter tip and balloon.

Specifically, the present invention provides a position-monitoring, flow-directed catheter, comprising a catheter having a distal artery portion for positioning in the pulmonary artery, a ventricular portion proximal to the distal portion for positioning in the right ventricle of the heart and an atrial portion proximal to the ventricular portion for positioning in the right atrium of the heart. The catheter has an inflatable balloon at the distal tip of the catheter, which when inflated, floats and carries the catheter through the right atrium and right ventricle of the heart into the pulmonary artery and into the wedge position. The catheter has an inflation lumen with a port opening at the balloon, a through lumen with a port opening at the distal tip of the catheter for measuring pressure, and a position-monitoring lumen with a port opening between the distal artery portion and the ventricular portion of the catheter. The position-monitoring port is adapted to be positioned in the outflow tract of the right ventricle proximal to the pulmonic valve of the heart. The proximal port of the position-monitoring lumen is connected to a pressure measurement apparatus, such as a pressure transducer.

The present invention also provides a method of monitoring the position of a balloon-tipped, flow-directed catheter by measuring the pressure at the position-monitoring port, continously or intermittently, as needed, before inflating the balloon to measure pulmonary wedge pressure. A right ventricular pressure reading indicates that the balloon has not advanced significantly and that wedge pressure can be measured safely by inflating the balloon. A pulmonary artery pressure reading indicates that the position-monitoring port has crossed the pulmonic valve and that the balloon has migrated distally to a position that may not be safe for inflating the balloon to measure wedge pressure. If a pulmonary artery pressure is obtained at the position-monitoring port, the catheter is gradually withdrawn until a right ventricular pressure is obtained; then the balloon can be safely inflated to measure wedge pressure in the pulmonary artery.

The present invention has other objects and features of advantage which will become apparent from and are set forth in more detail in the following Detailed Description of the Invention and the accompanying drawings.

## Brief Description of the Drawings

FIG. 1 is a diagrammatic view of a heart showing proper placement of the catheter of the present invention.

FIG. 2 is a general view of the catheter with parts broken away and parts in section.

FIG. 3 is an enlarged longitudinal, sectional view of the distal end of the catheter.

FIG. 4 is a fragmentary longitudinal, sectional view showing the injectate port of the catheter of the present invention.

FIG. 5 is a cross-sectional view along line 5-5 in FIG. 2.

FIG. 6 is a fragmentary longitudinal, sectional view showing the thermistor located in the distal portion of the catheter and the position-monitoring port located between the distal portion and the ventricular portion of the catheter of the present invention.

## Detailed Description of the Invention

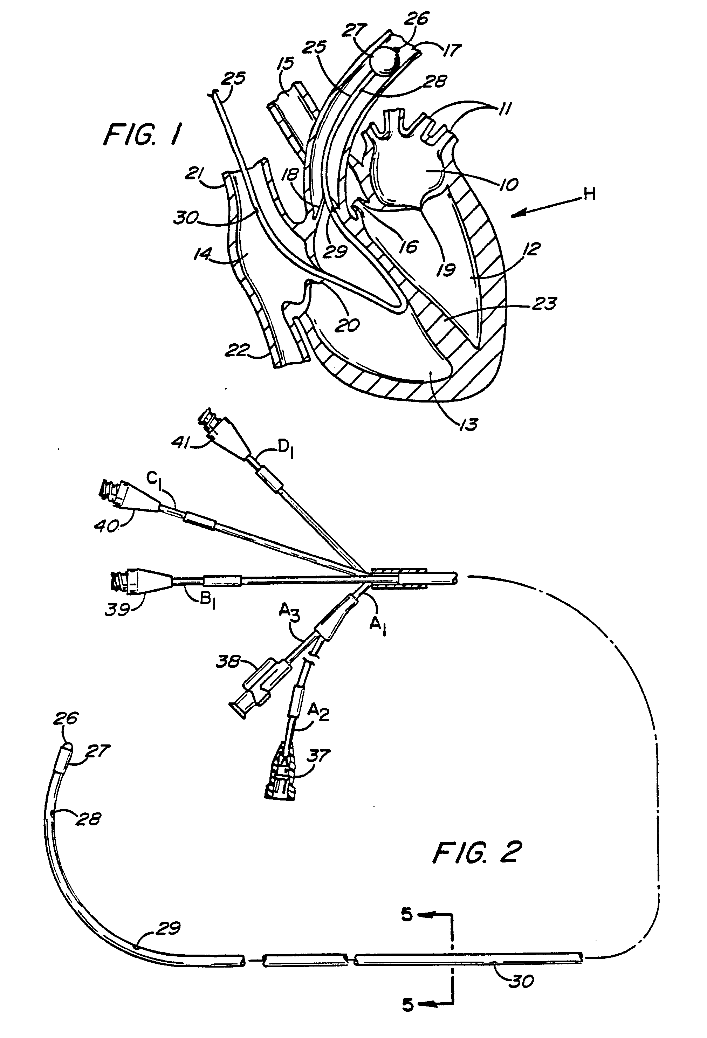

Referring to FIG. 1, the human heart is depicted with the catheter of the present invention properly inserted so that the position-monitoring port is in the outflow tract of the right ventricle, proximal to the pulmonic valve, and the balloon is in a safe position in the pulmonary artery for inflation to obtain pulmonary wedge pressure.

The heart has a left atrium 10, a left ventricle 12, a right ventricle 13, and a right atrium 14. The septum 23 separates the left and right ventricles of the heart. Unoxygenated blood is returned to the right atrium 14 through the superior vena cava 21 or the inferior vena cava 22 to the right atrium 14. The right atrium 14 pumps the blood through the tricuspid valve 20 to the right ventricle 13 which in turn pumps the blood through the pulmonic valve 18 to the pulmonary artery 17. The pulmonary artery 17 carries the blood to the lungs for oxygenation. The pulmonary veins 11 return the oxygenated blood to the heart through the left atrium 10 which pumps the blood through the mitral valve 19 to the left ventricle 12 which pumps the blood through the aortic valve 16 into the aorta 15. The aorta 15 carries the oxygenated blood to the body.

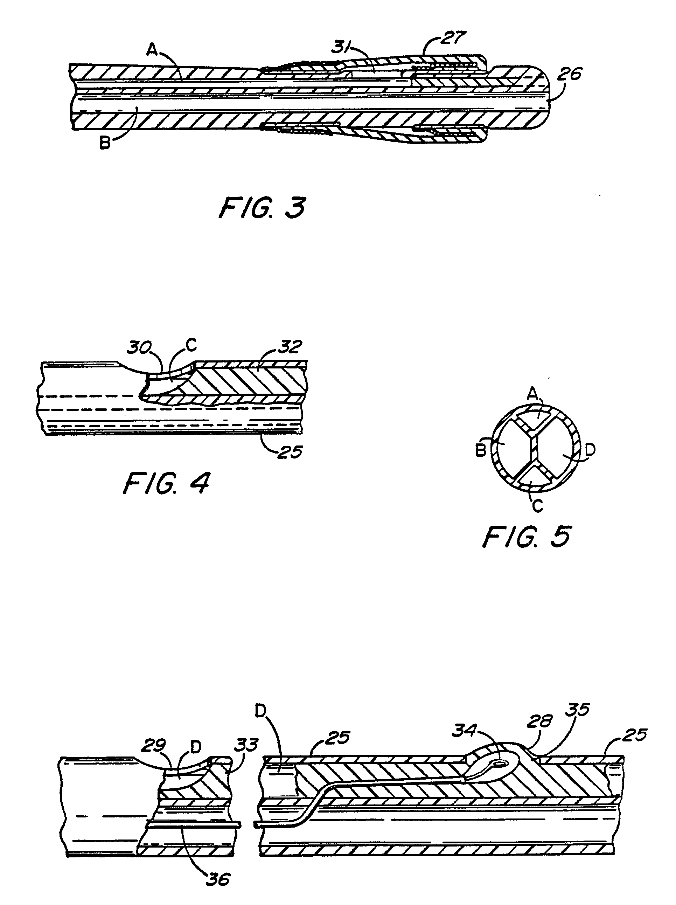

The catheter 25 of the present invention, depicted in FIG. 1, preferably contains four lumens A, B, C and D. See FIG. 5 for a cross-section of the catheter and the four lumens. Lumen A is a balloon inflation lumen for inflating the balloon 27 through the port opening 31, as shown in FIG. 3. Balloon 27 is a flotation balloon preferably made according to U.S. Patent No. 3,634,924, issued January 18, 1972, in the name of Blake et al., for a Method of Making a Multilumen Balloon Catheter.

Lumen B is a through lumen with a port opening 26 at the distal tip of the catheter as shown in Figures 1, 2 and 3. Lumen C is the injectate lumen with a port opening at 30, as shown in Figures 1, 2 and 4. Lumen C is plugged on the distal side of the port opening 30 with an adhesive plug 32 or the like, as shown in Figure 4. Lumen D is the position-monitoring lumen with a port opening at 29, as shown in Figures 1, 2 and 6. Lumen D is plugged on the distal side of the port opening 29 with an adhesive plug 33 or the like, as shown in Figure 6. A thermistor 34 is potted in a plug 35 of a polymeric material or an adhesive material or the like, at an exterior opening 28 in lumen D. Adhesive plug 35 also closes lumen D at this point. Because of the plug at 33, the thermistor lead wires 36 are fed from lumen D to lumen A, the balloon inflation lumen, and through lumen A to contact pins in an electrical connector plug 37, shown in Figure 2. If more that four lumens are present in the catheter, the thermistor and lead wires could be in a lumen dedicated to the thermistor so that a crossover from one lumen to another would not be required.

The four lumens A, B, C and D communicate with proximal end connector tubes A-1, B-1, C-1 and D-1, respectively, as shown in Figure 2. Tube A-1 communicates with two branches A-2 and A-3. Tube A-2 leads to thermistor connector plug 37 and tube A-3 leads to connector 38 for connection to an inflation apparatus, such as an inflation syringe. Bacteria-filtered carbon dioxide is the preferred inflation medium because of its rapid absorption in the blood in the event of balloon rupture within the circulation. Air may be used but should never be used for balloon inflation in any situation where it may enter the arterial circulation.

Tube B-1 connects the through lumen B to a pressure measurement apparatus or pressure transducer at connector 39 in order to monitor pressures at the distal port 26. The distal port 26 is used to record pulmonary arterial pressures when the balloon is deflated and pulmonary wedge pressures when the balloon is inflated and in the pulmonary artery wedge position.

Tube C-1 and connector 40 connect injectate lumen C to a source of liquid or to various apparatus depending upon the desired use of lumen C. Lumen C can be used for multiple purposes, including right atrium pressure monitoring, blood sampling, injection or infusion of liquids for therapeutic or diagnostic purposes, and right atrial pacing by insertion of an electrode probe through lumen C and out port 30.

In most cases, lumen C is used to determine cardiac output by the thermodilution technique. A known amount of a chilled, sterile solution of known temperature is injected through a syringe at the injectate connector 40 and travels through lumen C to port 30 where it enters the bloodstream in the right atrium or a vena cava, depending on the position of the catheter. The resulting change in blood temperature is measured downstream in the pulmonary artery by the thermistor 34. From the temperature change, the cardiac output can be calculated in accordance with the well-known Fick principle.

Typically, in a catheter used for adult patients, the thermistor 34 is located in the distal portion of the catheter at approximately 2 to 6 cm, preferably 4 cm, from the distal tip, but can be located virtually anywhere along the length of the catheter as long as it is located downstream from the injectate port. Injectate port 30 is typically located in the atrial portion of the catheter at approximately 25 to 35 cm, preferably 30 cm, from the distal tip, but can be located anywhere along the length of the catheter as long as it is sufficiently upstream from the thermistor to permit measurements of the change in temperature of the bloodstream resulting from introduction of the injectate into the bloodstream. These port locations ensure that in most adult patients, the catheter ports will be positioned in the desired location of the patient's heart, that is, the right atrium for port 30 and the pulmonary artery for thermistor 34. For children or small adults, the port locations will necessarily vary.

Tube D-1 connects the position-monitoring lumen D to a pressure measurement apparatus, preferably a pressure transducer, at connector 41. The pressure at port 29 can then be monitored to determine the position of the distal tip or balloon. When the catheter is in the proper position, port 29 will be in the outflow tract of the right ventricle proximal to the pulmonic valve, as shown in Figure 1, and a right ventricular pressure measurement will be obtained.

If the distal tip or balloon has migrated distally into a narrower branch of the pulmonary artery, port 29 will have moved across the pulmonic valve into the pulmonary artery, and in such a case, a pulmonary arterial pressure will be obtained. A pulmonary artery pressure indicates that the catheter may not be in a safe position for inflation and should therefore be withdrawn gradually until a right ventricular pressure is obtained before the balloon is inflated to measure a pulmonary wedge pressure. Typically, the port 29 will be located at approximately 6 to 10 cm, preferably 8 cm, from the distal tip. This location in most adult patients will ensure that the port is just proximal to the pulmonic valve when the distal tip and balloon are in a safe position to obtain pulmonary wedge pressure. The port location will necessarily vary in catheters used for children and small adults.

A single pressure transducer can be used for measuring pressure through lumens B and D by installing a three-way stopcock on the pressure transducer. The stopcock can then be turned to monitor the pressure at either the distal port of lumen B or the position-monitoring port of lumen D.

A particular advantage of the present catheter is that it can be inserted at the patient bedside without fluoroscopic control. It can be inserted through a median basilic or deep brachial vein by a cutdown, but more often it is inserted percutaneously from the femoral, jugular or subclavian vein. As the catheter 25 is advanced, intravascular pressures are continuously monitored at the distal port 26. The balloon 27 is inflated when the catheter 25 has been advanced approximately to the junction of the superior vena cava and the right atrium, typically about 40 cm from the tip when the right arm vein is used, about 50 cm from the tip when the left arm is used, about 10 to 20 cm when the jugular or subclavian vein is used, and about 25 to 30 cm when the femoral vein is used.

When the balloon is inflated, the catheter is advanced further so that the balloon floats with the bloodstream through the right atrium and right ventricle into the pulmonary artery and then into the pulmonary wedge position. When pulmonary wedge pressure is initially obtained at the tip, the balloon is deflated and the position of the tip is checked by taking a pressure measurement at the position-monitoring port 29. A right ventricular pressure at 29 indicates that the balloon is in a safe position in the pulmonary artery for measuring pulmonary wedge pressure. If a pulmonary artery pressure is obtained, the catheter has been advanced too far distally into the pulmonary artery and should be withdrawn gradually until a right ventricular pressure reading is obtained at port 29. The position of the balloon should be checked by obtaining a pressure measurement at port 29 before each pulmonary wedge pressure measurement is taken, as the balloon may have migrated distally even after proper placement initially. Evidence of the migration will be a pulmonary artery pressure reading instead of a right ventricular pressure reading.

While the preferred embodiment of the present invention has been described above, the catheter could be made with only three lumens if the physician desires only to monitor pressure at the distal tip and not to determine cardiac output. In that case, the catheter would have a through lumen for pressure measurement at the distal tip, an inflation lumen for inflating the balloon and a position-monitoring lumen for monitoring the pressure at port 29.

In some applications, it may be desirable to have an additional lumen and port for continuous infusion of liquid in addition to a lumen for injecting liquid to determine cardiac output by the thermodilution method. Such a catheter would be made according to the present invention but would have five lumens. A lumen for infusing liquid, a balloon inflation lumen with the thermistor wires, an infusion lumen, which could also be utilized for cardiac pacing, an injectate lumen, a through lumen, and a position-monitoring lumen.

In some applications, the present invention may also include an additional lumen for cardiac pacing. The catheter would be identical to the catheter described in the preferred embodiment but would have an additional lumen with a port in the ventricular portion for right ventricular pacing. The injectate port 30 could then be used for right atrial pacing. It may also be desirable to include ventricular and atrial coil electrodes on the catheter itself as disclosed and described in U.S. Patent No. 3,995,623, Issued December 7, 1976 in the name of Blake, et al, for a Multipurpose Flow-Directed Catheter.

The catheter and method of the present invention contribute significantly to the safety of hemodynamic monitoring with a balloon-tipped, flow-directed catheter. In the past, the physician could monitor tip placement only by fluoroscopy and chest X-rays. Some physicians attempted to monitor tip placement by taking pressure measurements at the distal tip. For instance, if a wedge pressure reading was obtained when the balloon was deflated or inflated with volumes substantially less than the recommended volume printed on the catheter, then the physician would assume that the catheter should be withdrawn slightly. This method proved to be ineffective. The present invention provides a much more precise, reliable and safe method of monitoring placement of the catheter tip and obtaining safe pulmonary wedge pressure readings.

## Claims

1. A position-monitoring, flow-directed catheter, comprising:

a) a catheter having a distal artery portion and a distal tip, a ventricular portion proximal to the distal portion, and an atrial portion proximal to the ventricular portion;

b) an inflatable balloon located at the distal tip of the distal portion of the tube, adapted to float with the bloodstream and carry the tube through the right atrium and right ventricle of the heart into the pulmonary artery and into a wedge position when the balloon is inflated;

c) an inflation lumen in the catheter with a port opening communicating with the balloon's interior;

d) a through lumen in the catheter with a port opening at the distal end of the catheter; and

e) a position-monitoring lumen in the catheter adapted to be connected to pressure measurement apparatus, said lumen having a port opening between the distal portion and the ventricular portion of the catheter adapted to be positioned in the outflow tract of the right ventricle proximal to the pulmonic valve of the heart, wherein the position of the balloon in the pulmonary artery can be monitored by measuring the pressure at the position-monitoring port.

2. A catheter according to Claim 1, wherein the through lumen adapted to be connected to a pressure measurement apparatus for measuring pressure at the distal tip of the catheter.

3. A catheter according to Claim 2, wherein the through lumen and the position-monitoring lumen are adapted to be connected to a single pressure measurement apparatus with port selection means so that pressure readings can be taken selectively at either the distal tip port or the position-monitoring port.

4. A catheter according to Claim 1, wherein the catheter has an additional lumen and port suitable for infusion or injection of liquids into the bloodstream, for blood sampling, for pressure monitoring, or for insertion of an electrode probe for cardiac pacing.

5. A catheter according to Claim 1, wherein the catheter has temperature measuring means.

6. A catheter according to Claim 1, wherein the catheter has temperature measuring means in the distal portion of the catheter proximal to the balloon and an additional lumen having a port opening proximal to the temperature measuring means for injecting liquid into the bloodstream.

7. A catheter according to Claim 6, wherein the additional lumen port opening is in the atrial portion of the catheter adapted to be positioned in the right atrium.

8. A catheter according to Claim 6, wherein lead wires from the temperature measuring means are fed through the inflation lumen to a proximal end connector.

9. A catheter according to Claim 1, wherein the catheter has temperature measuring means in the distal portion of the catheter proximal to the balloon and two additional lumens having port openings, one for injecting liquids and one for infusing liquids.

10. A catheter according to Claim 9, wherein the lumen for injecting liquids has a port opening proximal to the temperature measuring means.

11. A catheter according to Claim 1, wherein the catheter has a pacing lumen with a port opening in the atrial or ventricular portion of the catheter adapted to be positioned in the right artrium or right ventricle for insertion of a pacing electrode through the pacing lumen into the right atrium or right ventricle for cardiac pacing.

12. A catheter according to Claim 1, wherein the catheter has a pacing lumen with a port opening in the ventricular portion of the catheter adapted to be positioned in the right ventricle for insertion of a pacing electrode into the right ventricle for cardiac pacing and a lumen with a port opening in the atrial portion of the catheter adapted to be positioned in the right atrium suitable for injecting or infusing liquid into the right atrium, for blood sampling, for right atrial pressure monitoring, or for insertion of a pacing electrode into the right atrium for cardiac pacing.

13. A method of monitoring the position of a flow-directed catheter, said catheter having a distal artery portion and a distal tip, a ventricular portion proximal to the distal portion and an atrial portion proximal to the ventricular portion, an inflatable balloon located at the distal tip of the distal portion, adapted to float with the bloodstream and carry the catheter through the right atrium and right ventricle of the heart into the pulmonary artery and into a wedge position when the balloon is inflated, an inflation lumen in the catheter with a port opening communicating with the balloon's interior, a through lumen in the catheter adapted to be connected to pressure measurement apparatus and having a port opening at the distal tip of the catheter for measuring pressure, and a position-monitoring lumen in the catheter adapted to be connected to pressure measurement apparatus and having a port opening between the distal portion and the ventricular portion of the catheter, adapted to be positioned in the outflow tract of the right ventricle, proximal to the pulmonic valve of the heart; the method comprising

a) positioning the inflated balloon in a pulmonary wedge position and then deflating the balloon;

b) before inflating the balloon to measure pulmonary wedge pressure at the distal port, monitoring the position of the balloon by measuring the pressure at the position-monitoring port, a right ventricular pressure reading indicating that the balloon is in a safe position in the pulmonary artery for measuring pulmonary wedge pressure, a pulmonary artery pressure reading indicating that the position-monitoring port has crossed the pulmonic valve and that the balloon has migrated distally to a potentially unsafe position for measuring wedge pressure;

c) if a pulmonary artery pressure reading is obtained, withdrawing the catheter until a right ventricular pressure reading is obtained; and

d) then, inflating the balloon to obtain a safe pulmonary wedge pressure reading at the distal port.

FIG. 1

FIG. 2

FIG. 3

FIG. 4

FIG. 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y,D | US-A-3995623 (L.W.BLAKE ET AL.) <br> * column 2, line 53 - column 3, line 48 * | 1, 2, 5, 13 | A61B5/0215 <br> A61B5/028 |
| A,D | * column 4, line 14 - column 5, line 18; figures 1-6 * | 4, 7, 8, 11, 12 | G01L19/00 |
| Y | US-A-4003370 (T.EMIL ET AL.) <br> * column 8, lines 5 - 68; figures 5-8 * | 1, 2, 5, 13 | |
| A | US-A-4718423 (A.F.WILLIS ET AL.) <br> * abstract * <br><br> * column 6, lines 4 - 60 * <br> * column 7, lines 3 - 40; figures * | 1, 2, 4-6, 8-12 | |
| A,D | US-A-4696304 (A.K.CHIN) <br> * abstract * <br><br> * column 3, lines 15 - 55 * <br> * column 4, lines 3 - 66 * <br> * column 5, lines 1 - 18; figures * | 1, 2, 4-6, 10, 13 | |
| A | US-A-4733566 (Y.MORIUCHI ET AL.) <br> * abstract * <br> * column 1, lines 40 - 60 * <br> * column 2, lines 22 - 64 * <br> * column 3, line 52 - column 4, line 12; figures 1, 10 * | 1, 3 | **TECHNICAL FIELDS SEARCHED (Int. Cl.5 )** <br><br> A61B <br> G01L |

The present search report has been drawn up for all claims

| Place of search | Date of completion of the search | Examiner |
|---|---|---|
| THE HAGUE | 10 JANUARY 1990 | RIEB K.D. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P0401)